# EUROPEAN PATENT SPECIFICATION

(11) **EP 2 868 309 B1**
(45) Date of publication and mention of the grant of the patent: **06.03.2019**
(21) Application number: 13191128.1
(22) Date of filing: 31.10.2013
(51) Int. Cl.: A61K 8/34, A61Q 5/06, A61K 8/81, A61K 8/898

(54) **Hair styling composition**
Haarstylingzusammensetzung
Composition de coiffage

(43) Date of publication of application: 06.05.2015
(73) Proprietor: Kao Corporation, Chuo-ku Tokyo 103-8210 (JP)
(72) Inventor: Cajan, Christine, 64297 Darmstadt (DE); Weichaus, Dirk, 64297 Darmstadt (DE); Punsh, Britta, 64297 Darmstadt (DE)
(74) Representative: Hoffmann Eitle

(56) References cited:
- EP-A1- 0 863 172
- EP-A1- 2 022 479
- EP-A1- 2 502 615
- EP-A2- 1 161 934
- WO-A1-2012/072774
- US-A1- 2012 207 696
- DATABASE GNPD [Online] MINTEL; December 2006 (2006-12), Anonyme: "Modeler Shaping Spray", XP002722289, Database accession no. 626163

## Description

The present invention is related to hair styling composition.

Aerosol hair styling compositions have been widely used either as a spray, aerosol or non-aerosol, or as foam. In principal, they comprise hair fixing polymers in an aqueous or aqueous alcoholic or alcoholic medium together with a propellant in case of an aerosol composition. Many fixing polymers have been suggested in the literature either synthetic or natural origin. Natural polymers so far suggested do not satisfy the expectations of consumers in terms of hair feel and hold of hair style for a long time and, therefore, overwhelmingly synthetic polymers are used.

Hair sprays providing high hold are usually based on acrylate polymers. The acrylate polymers offer higher hold and are ideally suitable for hair sprays. The acrylate polymers used up until now do not present sufficient thermoplastic behaviour because they have high glass transition temperature (Tg) and therefore they are not suitable for using with heat aided hair styling for example in combination with heat irons. The hair treated with such polymers becomes very stiff, rough and the hair feels artificial.

On the other hand, thermoplastic polymers with low Tg that are ideal for setting hair in a new style using heat are not suitable for hair sprays as they do not provide the hair with high holding power.

Thus, the currently available technologies provide hair with high hold but artificial, hard feeling and rough hair surface and are not designed for using heat aided styling tools.

EP 0 863 172 concerns specific organopolysiloxanes composed of an organopolysiloxane segment and a poly(N-acylalkyleneimine) segment which is bonded to at least one silicone atom of said organopolysiloxane segment via an alkylene group. These organopolysiloxanes are soluble or dispersible in water and can form a film with an excellent texture. Thus, they can be appropriately employed as additives for cosmetic products.

EP 2 022 479 relates to a hair styling composition for keratin fibres comprising at least one film forming polymer and at least one arylated silicone, preferably trimethyl pentaphenyl trisiloxane.

EP 1 161 934 describes a hair-spray composition for an aerosol container equipped with a tubular extension, comprising: (a) film former(s), (b) solvent(s) of boiling point 30-95 degrees C under normal pressure, (c) propellant(s) selected from hydrocarbons, 1,2-difluoroethane, dimethyl ether, carbon dioxide and/or nitrogen and optionally (d) water.

EP 2 502 615 is directed to a hair styling method, including the steps of: applying a hair cosmetic composition containing a specific poly(N-acylalkyleneimine) modified organopolysiloxane to the hair; forming the hair style at a hair temperature of 50°C or higher; and subsequently cooling the hair temperature to lower than 50°C to fix the formed hair style.

WO 2012/072774 relates to acrylic emulsion polymers as well as to their use in hair care preparations such as in particular in hair styling preparations.

US 2012/207696 concerns acrylic polymer beads having very low residual monomer content as well as their use in hair care preparations such as a styling agent.

There is a great need for new technologies providing heat aided hair styling with strong hold, letting hair still feel natural with highest style durability and style should still hold after combing through the hair.

The inventors of the present invention have unexpectedly found out that one or more of the above mentioned problems is (are) solved with a composition comprising an acrylate copolymer having relatively low glass transition temperature and a silicone polymer. It has been surprisingly found out that the combination of the two polymers enables the formation of flexible smooth polymer film on the hair surface which makes hot shaping with highest durability possible when treated at a temperature higher than the glass transition temperature of the polymer.
Thus, the first object of the present invention is an aqueous, alcoholic or aqueous-alcoholic composition for hair comprising at least one acrylate copolymer with a glass transition temperature (Tg) in the range of 80 to 120°C measured with differential scanning calorimeter (DSC) at a heating speed of 2°C/min and a silicone polymer grafted with polyethyl oxazoline units, the acrylate copolymer being selected from terpolymers of butyl methacrylate, methacrylic acid and ethyl acrylate and tetra polymers of butyl methacrylate, methacrylic acid, ethyl acrylate and ethyl methacrylate.
The second object of the present invention is a process for treating hair wherein the composition of the present invention is applied onto hair and optionally hair is styled with an iron at a surface temperature in the range of 130 to 230°C.
The third object of the present invention is the use of the composition of the present invention for styling hair, preferably styling hair with an iron at a surface temperature in the range of 130 to 230°C.
The fourth object of the present invention is a kit for treating hair comprising one or more compositions and optionally one or more tools, preferably with heating capability wherein it comprises the composition of the present invention.
Composition of the present invention comprises at least one acrylate copolymer with a glass transition temperature (Tg) in the range of 80 to 120°C measured with differential scanning calorimeter (DSC) at a heating speed of 2°C/min. The concentration of the polymer is in the range of 0.1 to 20%, preferably 0.2 to 15, more preferably 0.25 to 12.5 and most preferably 0.5 to 10% by weight calculated to the total composition excluding propellant, if present.
Preferably the acrylate copolymer has a Tg in the range of 85 to 115°C, more preferably 90 to 110°C and most preferably 95 to 110°C measured with differential scanning calorimeter (DSC) at a heating speed of 2°C/min.

The acrylate copolymers are terpolymers of butylmethacrylate, methacrylic acid and ethyl acrylate which is available under the trade name Tilamar Fix A140 from DSM and tetra polymers of butyl methacrylate, methacrylic acid, ethyl acrylate and ethyl methacrylate which is commercially available under the trade name Tilamar Fix A1000 form the company DSM.
The most preferred is a tetrapolymer of butyl methacrylate, methacrylic acid, ethyl acrylate and ethyl methacrylate and is commercially available under the trade name Tilamar Fix A1000 form the company DSM.
In order to improve and/or make the acrylate polymer soluble in the cosmetically acceptable medium, the polymer is neutralised with the known bases. Preferably the acrylate copolymer is 70% to 100% neutralised and more preferably 100% neutralised. For this purpose any of the know bases may be used however alkanolamines are preferred and the most preferred is amino methyl propanol in particular 2-methyl-2-amino propanol.
Compositions of the present invention comprise a silicone polymer grafted with polyethyl oxazoline units. Such polymers are known with their CTFA adopted name Polysilicone-9 and commercially available from Kao Corporation-Japan under the trade name Elastomer OS. Concentration of grafted silicone polymer is in the range of 0.01 to 5%, preferably 0.05 to 2.5%, more preferably 0.1 to 2% and most preferably 0.1 to 1% by weight calculated to the total composition excluding propellant, if present.
Preferred organopolysiloxane polymers are those of the type disclosed in EP-A 640 643, in particular optionally quaternized aminoalkyl, in particular aminopropyl dimethyl polysiloxane/polyethyl oxazoline copolymers of the formula 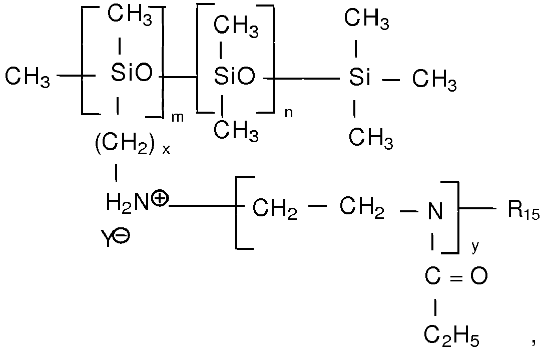 wherein m and n each are numbers from 20 to 10,000, in particular 50 to 7,000, especially 100 to 5,000, x is a number between 1 and 5, preferably 3, and y is a number from 5 to 30, R₁₅ is a C₁-C₁₂-alkyl or aryl group, in particular a methyl, ethyl or benzyl group, and Y⁻ is an anion.

Compositions of the present invention may comprise additionally one or more other film forming polymers preferably at a concentration not exceeding the concentration of the acrylate copolymers. In the more preferred embodiment, the additional one or more film forming polymer may be included in the compositions of the present invention up to a concentration which does not bring the Tg of the polymer mixture outside of the temperature ranges defined above.

Within the meaning of the present invention one or more film forming polymer is selected from the anionic, non-ionic, cationic and/or amphoteric or zwitterionic ones, especially the non-ionic, anionic and amphoteric are preferred and the cationic should be excluded as soon as an incompatibility occurred with the acrylate copolymers.

Suitable non-ionic polymer is first of all vinylpyrrolidon polymers either homopolymers or copolymers with, especially, vinylacetate. Those are known with the trade name "Luviskol" as homopolymers Luviskol K 30, K 60 or K 90 as well copolymers Luviskol VA 55, VA 64, Plus from BASF AG and Advantage LC-E from ISP.

Natural non-ionic polymers are as well suitable for the composition of the present invention. Those are such as cellulose, chitosan, guar gum, neutralised shellac and their derivatives.

As amphoteric polymers which can be used alone or in mixture with at least one additional nonionic polymer, reference is here made in particular to copolymers of N-octyl acrylamide, (meth)acrylic acid and tert.-butyl aminoethyl methacrylate of the type "Amphomer®"; copolymers from methacryloyl ethyl betaine and alkyl methacrylates of the type "Yukaformer®", e.g.. the butyl methacrylate copolymer "Yukaformer® Am75"; copolymers from monomers containing carboxyl groups and sulfonic groups, e.g.. (meth)acrylic acid and itaconic acid, with monomers such as mono- or dialkyl aminoalkyl (meth)acrylates or mono- or dialkyl aminoalkyl (meth)-acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from US-A 3,927,199.

Suitable anionic polymers alone or in combination with non-ionic polymers are vinyl - alkyl ether, in particular methyl vinyl ether/maleic acid copolymers, obtained by hydrolysis of vinyl ether/maleic anhydride copolymers, distributed under the trade name "Gantrez® AN or ES". These polymers may also be partly esterified, as for example, "Gantrez® ES 225" or "ES 435", the ethyl ester of an ethyl vinyl ether/maleic acid copolymer, or the butyl or isobutyl ester thereof.

Further useful anionic polymers are in particular vinyl acetate/crotonic acid or vinyl acetate/vinyl neodecanoate/crotonic acid copolymers of the type "Resyn®"; sodium acrylate/vinyl alcohol copolymers of the type "Hydagen® F", sodium polystyrene sulfonate, e.g.. "Flexan® 130"; ethyl acrylate/acrylic acid/N-tert.-butyl acrylamide copolymers of the type "Ultrahold®"; vinyl pyrrolidone/vinyl acetate/itaconic acid copolymers, acrylic acid/acrylamide copolymers or the sodium salts thereof.

Further suitable anionic polymers are Acrylate copolymers available under trade name Salcare SC 81, PEG/PPG 25/25 dimethicone / acrylate copolymer available under trade name Luviflex Silk from BASF, Acrylates/t-butylacrylamide copolymer available under trade name Ultrahold Strong, Advantage LC-E which is vinylcaprolactam/PVP/dimethylaminoethylmethacrylate copolymer and VA/crotonates copolymer available under trade name Luviset CA 66.

Composition of the present invention may comprise, although less preferred, cationic polymers alone or in combination with non-ionic polymer. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, those cationic polymers known with their CTFA category name Polyquaternium may as well be added into the compositions of the present invention. Typical examples of those are Polyquaternium 6, Polyquaternium 7, Polyquaternium 10, Polyquaternium 11, Polyquaternium 16, Polyquaternium 22 and Polyquaternium 28, Polyquaternium 30, Polyquaternium 37, Polyquaternium 36, Polyquaternium 46, Polyquaternium 24, Polyquaternium 67, and Polyquaternium 72.

In a further preferred embodiment of the present invention, the composition may comprise one or more synthetic or natural oil (s). In principal, any oil allowed for cosmetic use is suitable for the compositions of the present invention.

Oils are those of synthetic and of natural ones. Natural oils are in principal any triglyceride suitable for cosmetic use. Non-limiting examples are avocado oil, coconut oil, palm oil, sesame oil, peanut oil, whale oil, sunflower oil, almond oil, peach kernel oil, wheat germ oil, macadamia nut oil, night primrose oil, jojoba oil, castor oil, or also olive oil, soya oil, and the derivatives thereof. Mineral oils such as paraffin oil and petrolatum are suitably contained within the scope of the present invention, It should as well be noted that compositions of the present invention can contain mixture of one or more natural oils and mineral oil.

Further, suitable synthetic oil components are in particular fatty alcohol fatty acid esters such as isopropyl myristate, palmitate, stearate and isostearate, oleyl oleate, isocetyl stearate, hexyl laurate, dibutyl adipate, dioctyl adipate, myristyl myristate, oleyl erucate, polyethylene glycol and polyglyceryl fatty acid esters, cetyl palmitate, etc.

Synthetic ones are those of silicone oils. Here again any silicone oil either volatile and/or non-volatile is suitable for the compositions of the present invention. Preferred silicone oils are non-volatile silicone oils known with their INCI name as dimethicone and dimethiconol. Volatile silicone oils such as cyclomethicones may be used in combination with non-volatile silicones and/or other wax and/or oils mentioned above. Commercially, they are available from various companies for example Dow Corning with the known DC series, Wacker Chemie and Toray silicones. All commercially available non volatile silicones are suitable in the compositions of the present invention. Examples to those are DC 200 series, DC1401, DC 1403, DC 1501 and DC 1503. Furthermore, aminated silicones such as amodimethicone and arylated silicones comprising at least one aryl group in its molecule such as phenyl methicone, phenyl trimethicone, diphenyl dimethicone, diphenylsiloxy phenyl trimethicone, tetramethyl tetraphenyl trisiloxane, triphenyl trimethicone, tetramethyl tetraphenyl trisiloxane and trimethyl pentaphenyl trisiloxane can be advantageously comprised in the compositions of the present invention.

Concentration of one or more oil in the compositions of the present invention is between 0.01 and 10%, preferably 0.05 and 7.5% more preferably 0.1 and 5% and most preferably 0.25 and 2.5% by weight calculated to total composition.

Compositions of the present invention may comprise one or more surfactant for solubilising of one or more ingredients not soluble in the medium used or especially for the foam aerosol composition for achieving required foam especially the styling polymer when present does not have such properties. Suitable ones are of anionic, non-ionic, amphoteric and cationic surfactants or their mixtures. Cationic surfactants may be comprised in the compositions as long as they do not cause any compatibility issues with the polymer mixtures.

As a rule any cationic surfactant is suitable for the compositions of the present invention. Preferably at least one cationic surfactant is selected from the compounds with the general formula 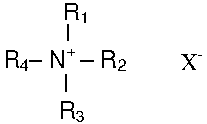 where R₁s a saturated or unsaturated, branched or non-branched alkyl chain with 8-22 C atoms or

R₅CONH(CH₂)ₙ

where R₅ is saturated or unsaturated, branched or non-branched alkyl chain with 7-21 C atoms and n has typical value of 1 - 4 or

R₆COO(CH₂)ₙ

where R₆ is saturated or unsaturated, branched or non-branched alkyl chain with 7-21 C atoms and n has typical value of 1 - 4, and
and R₂, R₃ and R₄ are independent from each other H or lower alkyl chain with 1 to 4 carbon atoms or ethoxy or propoxy group with number of ethoxy or propoxy groups varying in the range of 0 to 4, and X is chloride, bromide or methosulfate.

Non-limiting examples to suitable cationic surfactants are cetyl trimethyl ammonium chloride, myristoyl trimethyl ammonium chloride, behentrimonium chloride, trimethyl cetyl ammonium bromide, stearyl trimethyl ammonium chloride, dimethyl stearyl ammonium chloride, stearamidopropyldimethyl ammonium chloride.

Suitable non-ionic surfactants are alkyl polyglucosides of the general formula

R₇-O-(R₈O)ₙO-Zₓ

wherein R₇ is an alkyl group with 8 to 18 carbon atoms, R₈ is an ethylene or propylene group, Z is a saccharide group with 5 to 6 carbon atoms, n is a number from 0 to 10 and x is a number between 1 and 5. Examples are decyl polyglucoside, cocoyl polyglucoside both are commercially available.

Further nonionic surfactant components are, for example, long-chain fatty acid mono- and dialkanolamides, such as coco fatty acid mono and di ethanolamide and myristic fatty acid mono and di ethanolamide.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}".

Further nonionic surfactants useful in the compositions according to invention are C₁₀-C₂₂-fatty alcohol ethoxylates. Suitable non-limiting examples are oleth-10, oleth-11, oleth-12, oleth-15, oleth-16, oleth-20, oleth-25, oleth-30, oleth-35, oleth-40, laureth-10, laureth-11, laureth-12, laureth-13, laureth-15, laureth-16, laureth-20, laureth-25, laureth-30, laureth-35, laureth-40, laureth-50, ceteth-10, ceteth-12, ceteth-14, ceteth-15, ceteth-16, ceteth-17, ceteth-20, ceteth-25, ceteth-30, ceteth-40, ceteth-45, cetoleth-10, cetoleth-12, cetoleth-14, cetoleth-15, cetoleth-16, cetoleth-17, cetoleth-20, cetoleth-25, cetoleth-30, cetoleth-40, cetoleth-45, ceteareth-10, ceteareth-12, ceteareth-14, ceteareth-15, ceteareth-16, ceteareth-18, ceteareth-20, ceteareth-22, ceteareth-25, ceteareth-30, ceteareth-40, ceteareth-45, ceteareth-50, isosteareth-10, isosteareth-12, isosteareth-15, isosteareth-20, isosteareth-22, isosteareth-25, isosteareth-50, steareth-10, steareth-11, steareth-14, steareth-15, steareth-16, steareth-20, steareth-25, steareth-30, steareth-40, steareth-50, steareth-80 and steareth-100. Additional examples of similar compounds can be found in the cosmetic ingredient dictionaries and cosmetic textbooks.

Further non-ionic surfactants within the meaning of the present invention are polyalkyleneglycol ether of fatty acid glyceride or partial glyceride with at least 30 polyalkylene units are with 30 to 1000, preferably 30 to 500, more preferably 30 to 200 and most preferably 40 to 100 polyethyleneglycol units. Examples to those are PEG-30 hydrogenated castor oil, PEG-35 hydrogenated castor oil, PEG-40 hydrogenated castor oil, PEG-45 hydrogenated castor oil, PEG-50 hydrogenated castor oil, PEG-55 hydrogenated castor oil, PEG-60 hydrogenated castor oil, PEG-65 hydrogenated castor oil, PEG-80 hydrogenated castor oil, PEG-100 hydrogenated castor oil, PEG-200 hydrogenated castor oil, PEG-35 castor oil, PEG-50 castor oil, PEG-55 castor oil, PEG-60 castor oil, PEG-80 castor oil, PEG-100 castor oil, PEG-200 castor oil. Additional examples of similar compounds can be found in the cosmetic ingredient dictionaries and cosmetic textbooks.

Further suitable non-ionic surfactants are monoglycerides such as glyceryl stearate, glyceryl palmitate, glyceryl myristate, glyceryl behenate.

As further surfactant component, the compositions according to the invention can also contain amphoteric or zwitterionic surfactants. Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate.

Further, suitable within the meaning of the present invention are anionic surfactants of the sulfate, sulfonate, carboxylate and alkyl phosphate type, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono-, di- or tri alkyl phosphates.

Additional anionic surfactants useful are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula

R₉-(OC₂H₄)ₙ-O-CH₂COOX,

wherein R₉ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

Concentration of one or more surfactant in the compositions according to present invention may be in the range of 0.01 to 5%, preferably 0.05 to 2.5% and more preferably 0.1 to 1% by weight calculated to total composition. Preferred surfactants are non-ionic, amphoteric and anionic ones. The most preferred is non-ionic surfactants.

The composition of the present invention may comprise polyols at a concentration of 0.1 to 15%, preferably 0.2 to 10%, more preferably 0.5 to 7.5% by weight calculated to the total concentration . The most preferred ones are glycerine, propylene glycols, butylene glycol and hexylene glycol.

The compositions according to the invention may also comprise further agents, such as proteins, for example bamboo protein, and protein hydrolyzates and polypeptides, e.g. keratin hydrolyzates, collagen hydrolyzates of the type "Nutrilan®" or elastin hydrolyzates, as well as, in particular vegetable, optionally cationized protein hydrolyzates, for example "Gluadin®".

Additional natural plant extracts can as well form part of the compositions of the present invention. Those are incorporated usually in an amount of about 0.01 % to about 5 %, preferably 0.05 % to 3.5 %, in particular 0.1 % to 2 % by weight, calculated as dry residue thereof to the total composition . Suitable aqueous (e.g. steam-distilled) alcoholic or hydro-alcoholic plant extracts known **per se** are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, coconut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.
Suitable trade products are, for example, the various "Extrapon®" products, "Herbasol^{R}", "Sedaplant^{R}" and "Hexaplant^{R}". Extracts and the preparation thereof are also described in "Hagers Handbuch der pharmazeutischen Praxis". 4th Ed..
Compositions of the present invention may comprise UV filters either for stabilization of the product colour and/or for protection of hair from environmental influences such as loss of elasticity, loss of hair colour (bleaching effect of sun light). Suitable UV-absorbing substance are Polysilicone-15, 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2,4-dihydroxybenzophenone, 2,2',4,4'-tetrahydroxy- benzophenone, 2-hydroxy-4-methoxybenzophenone and its 5-sulfonic acid or the sodium salt thereof, 2,2'-dihydroxy-4,4'-dimethoxybenzophenone, 2-hydroxy-5-chlorobenzophenone, 2,2'-dihydroxy-4-methoxybenzophenone, 2,2'-dihydroxy-4,4'-dimethoxy-5,5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof and/or 3-(4'-methyl benzylidene)-DL-campher, and/or polysilicone-15.

The suitable amount of the UV-absorber ranges from about 0.01 % to 1% by weight, calculated to the total composition . Attention should be paid to the stability and solubility especially when using UV filter as salts, e.g. anionic UV filter salts.

In a further embodiment of the present invention, the compositions comprise at least one direct dye for colouring hair. Suitable direct dyes are cationic, anionic, neutral dyes and their mixtures as available commercially from various suppliers and used mainly in semi-permanent hair coloration.

Non-limiting examples to cationic dyes are Basic Blue 6, Basic Blue 7, Basic Blue 9, Basic Blue 26, Basic Blue 41, Basic Blue 99, Basic Brown 4, Basic Brown 16, Basic Brown 17, Basic Orange 31, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87.

Further suitable direct dyes are anionic dye. Suitable non-limiting examples are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Further suitable dyes for colouring hair within the meaning of the present invention are those of neutral nitro dyes. Suitable non-limiting examples are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.
Plant dyestuffs may also be used as hair colorant within the meaning of the present invention for example henna (red or black), alkanna root, laccaic acid, indigo, logwood powder, madder root and rhubarb powder, etc.
It should be noted that the above dyestuffs are also suitable for use in mixture. When using direct dyes of various categories, their compatibility must be checked.
The above mentioned dyestuffs are also used especially the anionic ones for product colouring at reduced concentrations.
Concentration of direct dyes in the compositions of the present invention is within the range of 0.001 to 5%, preferably 0.01 to 3% and more preferably 0.05 to 2%, and most preferably 0.1 to 1% by weight calculated to total composition, calculated to total composition.
In case an aerosol type of styling product is designed then composition of the present invention comprises additionally at least one propellant.

Compositions of the present invention may comprise at least one propellant at a concentration of 5 to 60%, preferably 5 to 50% more preferably 10 to 50% by weight calculated to total composition. Suitable propellants are lower alkanes such as n-butane, i-butane, propane, butane or their mixtures, as well as dimethylether (DME) either alone or in mixture with lower alkanes. Further suitable propellants are fluorinated hydrocarbons such as 1,1-difluoro ethane or tetrafluoroethane or their mixtures with each other, carbon dioxide and nitogen or their mixtures with the above mentioned propellants. Preferred are lower alkanes such as propane, butane, n-butane, i-butane, and their mixtures and their mixture with DME at a alkane to DME weight ratio 10:1 to 1:10.

Furthermore compositions of the present invention can comprise all substances customarily found in such preparations. Examples of such substances are complexing agents, preservatives, fragrances, etc.

The following examples are to illustrate the invention but not to limit.

In all examples below the acrylate polymer used is 100% neutralised.

### Example 1 Pump spray

| | % by weight |
|---|---|
| Tilamar Fix A1000 | 3.00 |
| Polysilicone-9 | 0.25 |
| 2-methyl-2-amino propanol | q.s. to pH 7.0 |
| Fragrance | q.s. |
| PEG-60 hydrogenated castor oil | 0.5 |
| Water | 50.00 |
| Ethanol | q.s to 100 % |

The above composition was prepared by dissolving - dispersing all ingredients one by one in water and after adjusting the pH to 7.0 ethanol and fragrance mixture was assed to the composition.

The above composition was applied on a hair streak which was cleansed with a commercial shampoo composition and dried prior to application. The streak was left to dry and combed through. The streak was smooth, softer and felt natural upon touching.

The streak was shaped with a curling iron having a surface temperature of 180°C and it kept it shape for long time and felt at the same time softer and natural upon touching. Afterwards the same streak was straighten with a flat iron at 180°C and still kept its softer and natural feeling upon touching. Even the straightened hair was easily brushed. The same streak was shaped again using the curling iron with a surface temperature of 180°C and it was observed that after stretching the streak to its full length, it spring back into its original shape indicating clearly that the streak has exceptionally good elasticity. After 3 hair styling cycles as described above the hair was easily brushed out and felt smooth and softer and natural upon touching.

In the similar way a streak treated with a composition as above comprising only Polysilicone-9 none of the above was possible to carry out. In the same way, when a streak treated with a composition comprising only Tilamar Fix A1000, it was also not possible to obtain a smooth brushable hair streak with the same softer and natural felling upon touching.

The examples below are within the scope of the present invention.

### Example 2

| | Pumpspray |
|---|---|
| | |
| | % |
| Ethanol | 70.0 |
| Tilamar Fix A1000 | 2.5 |
| Polysilicone-9 | 0.5 |
| VP/VA copolymer | 0.5 |
| 2-methyl-2-amino propanol | q.s. to pH 8.0 |
| Fragrance | q.s. |
| PEG-60 hydrogenated castor oil | 0.5 |
| Trimethyl pentaphenyl trisiloxane | 0.1 |
| Water | q.s to 100.0 |

### Example 3 Aerosol spray

| **Bulk** | % by weight |
|---|---|
| Tilamar Fix A1000 | 2.5 |
| Polysilicone-9 | 0.5 |
| Fragrance | 0.2 |
| Sodium hydroxide | q.s. to pH 7.9 |
| Alcohol | q.s. to 100 |

| **Aerosol:** | |
|---|---|
| Bulk of above | 50 % by weight |
| Dimethylether | 50 % by weight |

### Example 4 Aerosol spray

| **Bulk** | % by weight |
|---|---|
| PVP (Luviskol K 90) | 0.3 |
| Tilamar Fix A1000 | 2.5 |
| Polysilicone-9 | 0.5 |
| Phenyl trimethicone | 0.2 |
| Fragrance | 0.2 |
| PEG-60 hydrogentaed castor oil | 0.5 |
| Aminomethylpropanol | q.s. to pH 7.5 |
| Ethanol | q.s. to 100 |

| **Aerosol:** | |
|---|---|
| Bulk of above | 50 % by weight |
| 1,1-difluoro ethane | 50 % by weight |

### Example 5 Aerosol foam

| **Bulk** | % by weight |
|---|---|
| Ethanol | 10.0 |
| PVP (Luviskol K 90) | 0.3 |
| Tilamar Fix A1000 | 2.5 |
| Polysilicone-9 | 0.5 |
| Phenyl trimethicone | 0.2 |
| Fragrance | 0.2 |
| PEG-60 hydrogentaed castor oil | 0.5 |
| Cocamidopropyl betaine | 2.5 |
| Aminomethylpropanol | q.s. to pH 7.5 |
| Water | q.s. to 100 |

| **Aerosol:** | |
|---|---|
| Bulk of above | 90 % by weight |
| Propane butane | 10 % by weight |

### Example 5-A Quick breaking Root Lifting Aerosol foam

| | |
|---|---|
| Bulk of above | 60 % by weight |
| Propane butane | 15 % by weight |
| DME | 25 % by weight |

### Example 6 Color enhancing aerosol foam

| **Bulk** | % by weight |
|---|---|
| Ethanol | 10.0 |
| Tilamar Fix A1000 | 2.5 |
| Polysilicone-9 | 0.5 |
| Phenyl trimethicone | 0.2 |
| Fragrance | 0.2 |
| PEG-60 hydrogentaed castor oil | 0.5 |
| Cocamidopropyl betaine | 2.5 |
| Basic red 51 | 0.08 |
| Basid red 76 | 0.01 |
| Basic orange 31 | 0.01 |
| Basic yellow 87 | 0.01 |
| Aminomethylpropanol | q.s. to pH 7.5 |
| Water | q.s. to 100 |

| **Aerosol:** | |
|---|---|
| Bulk of above | 90 % by weight |
| Propane butane | 10 % by weight |

### Example 7 Foam with pump foamer

| | % by weight |
|---|---|
| Ethanol | 10.0 |
| Tilamar Fix A1000 | 2.5 |
| Polysilicone-9 | 0.5 |
| Phenyl trimethicone | 0.2 |
| Fragrance | 0.2 |
| PEG-60 hydrogentaed castor oil | 0.5 |
| Cocamidopropyl betaine | 2.5 |
| Aminomethylpropanol | q.s. to pH 7.5 |
| Water | q.s. to 100 |

### Example 8 Aerosol spray

| **Bulk** | % by weight |
|---|---|
| Tilamar Fix A1000 | 2.5 |
| Polysilicone-9 | 0.5 |
| Ethyl hexyl methoxycinnamate | 0.1 |
| Fragrance | 0.2 |
| Sodium hydroxide | q.s. to pH 7.9 |
| Alcohol | q.s. to 100 |

| **Aerosol:** | |
|---|---|
| Bulk of above | 50 % by weight |
| Dimethylether | 50 % by weight |

### Example 9 Aerosol spray

| **Bulk** | % by weight |
|---|---|
| Tilamar Fix A1000 | 2.5 |
| Polysilicone-9 | 0.5 |
| Isopropylpalmitate | 0.1 |
| Fragrance | 0.2 |
| Sodium hydroxide | q.s. to pH 7.9 |
| Alcohol | q.s. to 100 |

| **Aerosol:** | |
|---|---|
| Bulk of above | 50 % by weight |
| Dimethylether | 50 % by weight |

### Example 10 Aerosol spray

| **Bulk** | % by weight |
|---|---|
| Tilamar Fix A1000 | 2.5 |
| Polysilicone-9 | 0.5 |
| Almond oil | 0.1 |
| Fragrance | 0.2 |
| Sodium hydroxide | q.s. to pH 7.9 |
| Alcohol | q.s. to 100 |

| **Aerosol:** | |
|---|---|
| Bulk of above | 50 % by weight |
| Dimethylether | 50 % by weight |

### Example 11 Aerosol foam

| **Bulk** | % by weight |
|---|---|
| Ethanol | 10.0 |
| PVP (Luviskol K 90) | 0.3 |
| Tilamar Fix A1000 | 2.5 |
| Polysilicone-9 | 0.5 |
| Ethyl hexyl methoxycinnamate | 0.2 |
| Phenyl trimethicone | 0.2 |
| Fragrance | 0.2 |
| PEG-60 hydrogentaed castor oil | 0.5 |
| Cocamidopropyl betaine | 2.5 |
| Aminomethylpropanol | q.s. to pH 7.5 |
| Water | q.s. to 100 |

| **Aerosol:** | |
|---|---|
| Bulk of above | 90 % by weight |
| Propane butane | 10 % by weight |

### Example 12 Aerosol foam

| **Bulk** | % by weight |
|---|---|
| Ethanol | 10.0 |
| PVP (Luviskol K 90) | 0.3 |
| Tilamar Fix A1000 | 2.5 |
| Polysilicone-9 | 0.5 |
| Isopropyl palmitate | 0.2 |
| Phenyl trimethicone | 0.2 |
| Fragrance | 0.2 |
| PEG-60 hydrogentaed castor oil | 0.5 |
| Cocamidopropyl betaine | 2.5 |
| Aminomethylpropanol | q.s. to pH 7.5 |
| Water | q.s. to 100 |

| **Aerosol:** | |
|---|---|
| Bulk of above | 90 % by weight |
| Propane butane | 10 % by weight |

### Example 13 Aerosol foam

| **Bulk** | % by weight |
|---|---|
| Ethanol | 10.0 |
| PVP (Luviskol K 90) | 0.3 |
| Tilamar Fix A1000 | 2.5 |
| Polysilicone-9 | 0.5 |
| Almond oil | 0.2 |
| Phenyl trimethicone | 0.2 |
| Fragrance | 0.2 |
| PEG-60 hydrogentaed castor oil | 0.5 |
| Cocamidopropyl betaine | 2.5 |
| Aminomethylpropanol | q.s. to pH 7.5 |
| Water | q.s. to 100 |

| **Aerosol:** | |
|---|---|
| Bulk of above | 90 % by weight |
| Propane butane | 10 % by weight |

### Example 14 Foam with pump foamer

| | % by weight |
|---|---|
| Ethanol | 10.0 |
| Tilamar Fix A1000 | 2.5 |
| Polysilicone-9 | 0.5 |
| Ethyl hexyl methoxycinnamate | 0.2 |
| Fragrance | 0.2 |
| PEG-60 hydrogentaed castor oil | 0.5 |
| Cocamidopropyl betaine | 2.5 |
| Aminomethylpropanol | q.s. to pH 7.5 |
| Water | q.s. to 100 |

### Example 15 Foam with pump foamer

| | % by weight |
|---|---|
| Ethanol | 10.0 |
| Tilamar Fix A1000 | 2.5 |
| Polysilicone-9 | 0.5 |
| Isopropyl palmitate | 0.2 |
| Fragrance | 0.2 |
| PEG-60 hydrogentaed castor oil | 0.5 |
| Cocamidopropyl betaine | 2.5 |
| Aminomethylpropanol | q.s. to pH 7.5 |
| Water | q.s. to 100 |

### Example 16 Foam with pump foamer

| | % by weight |
|---|---|
| Ethanol | 10.0 |
| Tilamar Fix A1000 | 2.5 |
| Polysilicone-9 | 0.5 |
| Almond oil | 0.2 |
| Fragrance | 0.2 |
| PEG-60 hydrogentaed castor oil | 0.5 |
| Cocamidopropyl betaine | 2.5 |
| Aminomethylpropanol | q.s. to pH 7.5 |
| Water | q.s. to 100 |

### Example 17 Aerosol spray

| **Bulk** | % by weight |
|---|---|
| Tilamar Fix A1000 | 2.5 |
| Polysilicone-9 | 0.5 |
| Panthenol | 0.5 |
| Ethyl hexyl methoxycinnamate | 0.1 |
| Fragrance | 0.2 |
| Sodium hydroxide | q.s. to pH 7.9 |
| Alcohol | q.s. to 100 |

| **Aerosol:** | |
|---|---|
| Bulk of above | 50 % by weight |
| Dimethylether | 50 % by weight |

### Example 18 Aerosol spray

| **Bulk** | % by weight |
|---|---|
| Tilamar Fix A1000 | 2.5 |
| Polysilicone-9 | 0.5 |
| Glycerine | 0.2 |
| Isopropylpalmitate | 0.1 |
| Fragrance | 0.2 |
| Sodium hydroxide | q.s. to pH 7.9 |
| Alcohol | q.s. to 100 |

| **Aerosol:** | |
|---|---|
| Bulk of above | 50 % by weight |
| Dimethylether | 50 % by weight |

### Example 19 Aerosol spray

| **Bulk** | % by weight |
|---|---|
| Tilamar Fix A1000 | 2.5 |
| Polysilicone-9 | 0.5 |
| Propylene glycol | 0.1 |
| Almond oil | 0.1 |
| Fragrance | 0.2 |
| Sodium hydroxide | q.s. to pH 7.9 |
| Alcohol | q.s. to 100 |

| **Aerosol:** | |
|---|---|
| Bulk of above | 50 % by weight |
| Dimethylether | 50 % by weight |

### Example 20 Aerosol foam

| **Bulk** | % by weight |
|---|---|
| Ethanol | 10.0 |
| PVP (Luviskol K 90) | 0.3 |
| Tilamar Fix A1000 | 2.5 |
| Polysilicone-9 | 0.5 |
| Panthenol | 0.5 |
| Ethyl hexyl methoxycinnamate | 0.2 |
| Phenyl trimethicone | 0.2 |
| Fragrance | 0.2 |
| PEG-60 hydrogentaed castor oil | 0.5 |
| Cocamidopropyl betaine | 2.5 |
| Aminomethylpropanol | q.s. to pH 7.5 |
| Water | q.s. to 100 |

| **Aerosol:** | |
|---|---|
| Bulk of above | 90 % by weight |
| Propane butane | 10 % by weight |

### Example 21 Aerosol foam

| **Bulk** | % by weight |
|---|---|
| Ethanol | 10.0 |
| PVP (Luviskol K 90) | 0.3 |
| Tilamar Fix A1000 | 2.5 |
| Glycerine | 0.3 |
| Polysilicone-9 | 0.5 |
| Isopropyl palmitate | 0.2 |
| Phenyl trimethicone | 0.2 |
| Fragrance | 0.2 |
| PEG-60 hydrogentaed castor oil | 0.5 |
| Cocamidopropyl betaine | 2.5 |
| Aminomethylpropanol | q.s. to pH 7.5 |
| Water | q.s. to 100 |

| **Aerosol:** | |
|---|---|
| Bulk of above | 90 % by weight |
| Propane butane | 10 % by weight |

### Example 22 Aerosol foam

| **Bulk** | % by weight |
|---|---|
| Ethanol | 10.0 |
| PVP (Luviskol K 90) | 0.3 |
| Tilamar Fix A1000 | 2.5 |
| Polysilicone-9 | 0.5 |
| Propylene glycol | 0.2 |
| Almond oil | 0.2 |
| Phenyl trimethicone | 0.2 |
| Fragrance | 0.2 |
| PEG-60 hydrogentaed castor oil | 0.5 |
| Cocamidopropyl betaine | 2.5 |
| Aminomethylpropanol | q.s. to pH 7.5 |
| Water | q.s. to 100 |

| **Aerosol:** | |
|---|---|
| Bulk of above | 90 % by weight |
| Propane butane | 10 % by weight |

### Example 23 Foam with pump foamer

| | % by weight |
|---|---|
| Ethanol | 10.0 |
| Tilamar Fix A1000 | 2.5 |
| Polysilicone-9 | 0.5 |
| Propylene glycol | 0.2 |
| Ethyl hexyl methoxycinnamate | 0.2 |
| Fragrance | 0.2 |
| PEG-60 hydrogentaed castor oil | 0.5 |
| Cocamidopropyl betaine | 2.5 |
| Aminomethylpropanol | q.s. to pH 7.5 |
| Water | q.s. to 100 |

### Example 24 Foam with pump foamer

| | % by weight |
|---|---|
| Ethanol | 10.0 |
| Tilamar Fix A1000 | 2.5 |
| Polysilicone-9 | 0.5 |
| Glycerin | 0.2 |
| Isopropyl palmitate | 0.2 |
| Fragrance | 0.2 |
| PEG-60 hydrogentaed castor oil | 0.5 |
| Cocamidopropyl betaine | 2.5 |
| Aminomethylpropanol | q.s. to pH 7.5 |
| Water | q.s. to 100 |

### Example 25 Foam with pump foamer

| | % by weight |
|---|---|
| Ethanol | 10.0 |
| Tilamar Fix A1000 | 2.5 |
| Polysilicone-9 | 0.5 |
| Panthenol | 0.5 |
| Almond oil | 0.2 |
| Fragrance | 0.2 |
| PEG-60 hydrogentaed castor oil | 0.5 |
| Cocamidopropyl betaine | 2.5 |
| Aminomethylpropanol | q.s. to pH 7.5 |
| Water | q.s. to 100 |

## Claims

1. An aqueous, aqueous-alcoholic or alcoholic composition for hair **characterised in that** it comprises at least one acrylate copolymer with a glass transition temperature (Tg) in the range of 80 to 120°C measured with differential scanning calorimeter (DSC) at a heating speed of 2°C/min, the acrylate copolymer being selected from
- terpolymers of butyl methacrylate, methacrylic acid and ethyl acrylate; and
- tetra polymers of butyl methacrylate, methacrylic acid, ethyl acrylate and ethyl methacrylate;
and a silicone polymer grafted with polyethyl oxazoline units.

2. The composition according to claim 1 **characterized in that** it comprises at least one acrylate copolymer at a concentration in the range of 0.1 to 20%, preferably 0.2 to 15, more preferably 0.25 to 12.5 and most preferably 0.5 to 10% by weight calculated to the total composition excluding propellant, if present.

3. The composition according to claims 1 and 2 **characterized in that** the acrylate copolymer has a Tg in the range of 85 to 115°C, preferably 90 to 110°C and most preferably 95 to 110°C measured with differential scanning calorimeter (DSC) at a heating speed of 2°C/min.

4. The composition according to any of the preceding claims **characterised in that** at least one acrylate copolymer is a tetra polymer of butyl methacrylate, methacrylic acid, ethyl acrylate and ethyl methacrylate with a glass transition temperature in the range of 98 to 108°C measured with differential scanning calorimeter (DSC) at a heating speed of 2°C/min.

5. The composition according to any of the preceding claims **characterised in that** the silicone polymer grafted with polyethyl oxazoline units is of the formula 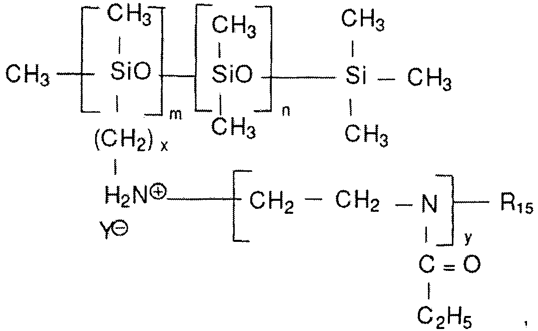 wherein m and n each are numbers from 20 to 10,000, in particular 50 to 7,000, especially 100 to 5,000, x is a number between 1 and 5, preferably 3, and y is a number from 5 to 30, R15 is a C1-C12-alkyl or aryl group, in particular a methyl, ethyl or benzyl group, and Y- is an anion, and preferably comprised at a concentration in the range of 0.01 to 5% by weight, calculated to the total composition excluding propellant, if present.

6. The composition according to any of the preceeding claims **characterised in that** it comprises additionally one or more other film forming polymers selected from anionic, non-ionic, cationic and amphoteric ones.

7. The composition according to any of the preceeding claims **characterised in that** it comprises one or more synthetic or natural oil.

8. The composition according to any of the preceeding claims **characterised in that** it comprises one or more surfactants selected from anionic, non-ionic, cationic and amphoteric ones.

9. The composition according to any of the preceeding claims **characterised in that** it comprises one or more polyols.

10. The composition according to any of the preceeding claims **characterised in that** it comprises one or more UV filter.

11. The composition according to any of the proceeding claims **characterised in that** it comprises one or more direct dye.

12. The composition according to any of the preceeding claims **characterised in that** it comprises one or more propellant, preferably at a concentration of 5 to 60% by weight calculated to the total composition and preferably selected from n-butane, iso-butane, propane, dimethylether, 1,1-difluoro ethane and their mixture.

13. Use of the composition as defined in any of the claims 1 to 12 for styling hair, preferably styling hair with an iron at a surface temperature in the range of 130 to 230°C.

14. Process for treating hair wherein a composition as defined in any of the claims 1 to 12 is applied onto hair and hair is styled with an iron with a surface temperature in the range of 130 to 230°C.

15. Kit for hair comprising one or more compositions and optionally one or more tools, preferably with heating capability, wherein it comprises a composition as defined in any of the claims 1 to 12.

## Patentansprüche

1. Wässrige, wässrig-alkoholische oder alkoholische Zusammensetzung für Haare, **dadurch gekennzeichnet, dass** sie zumindest ein Acrylat-Copolymer mit einer Glasübergangstemperatur (Tg) im Bereich von 80 bis 120°C, gemessen mit einem Differentialscanningkalorimeter (DSC) bei einer Erwärmungsgeschwindigkeit von 2°C/min, wobei das Acrylat-Copolymer ausgewählt ist aus
- Terpolymeren von Butylmethacrylat, Methacrylsäure und Ethylacrylat; und
- Tetrapolymeren von Butylmethacrylat, Methacrylsäure, Ethylacrylat und Ethylmethacrylat;
und ein mit Polyethyloxazolin-Einheiten gepfropftes Siliconpolymer umfasst.

2. Zusammensetzung gemäss Anspruch 1, **dadurch gekennzeichnet, dass** sie zumindest ein Acrylat-Copolymer in einer Konzentration im Bereich von 0,1 bis 20 %, vorzugsweise 0,2 bis 15, stärker bevorzugt 0,25 bis 12,5 und am meisten bevorzugt 0,5 bis 10 Gew.%, bezogen auf die gesamte Zusammensetzung, ausgenommen Treibmittel, falls vorhanden, umfasst.

3. Zusammensetzung gemäss den Ansprüchen 1 und 2, **dadurch gekennzeichnet, dass** das Acrylat-Copolymer eine Tg im Bereich von 85 bis 115°C, vorzugsweise 90 bis 110°C und am meisten bevorzugt 95 bis 110°C, gemessen mit einem Differentialscanningkalorimeter (DSC) bei einer Erwärmungsgeschwindigkeit von 2°C/min, umfasst.

4. Zusammensetzung gemäss einem der vorhergehenden Ansprüche, **dadurch gekennzeichnet, dass** zumindest ein Acrylat-Copolymer ein Tetrapolymer von Butylmethacrylat, Methacrylsäure, Ethylacrylat und Ethylmethacrylat mit einer Glasübergangstemperatur im Bereich von 98 bis 108°C, gemessen mit einem Differentialscanningkalorimeter (DSC) bei einer Erwärmungsgeschwindigkeit von 2°C/min, ist.

5. Zusammensetzung gemäss einem der vorhergehenden Ansprüche, **dadurch gekennzeichnet, dass** das mit Polyethyloxazolin-Einheiten gepfropfte Siliconpolymer die Formel: 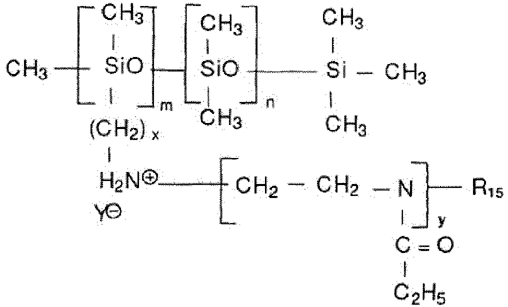 aufweist, worin m und n jeweils Zahlen von 20 bis 10.000, insbesondere 50 bis 7.000, im besonderen 100 bis 5.000, sind, x eine Zahl zwischen 1 und 5, vorzugsweise 3, ist und y eine Zahl von 5 bis 30 ist, R15 eine C₁₋₁₂-Alkyl- oder -Arylgruppe, insbesondere eine Methyl-, Ethyl- oder Benzylgruppe, ist und Y⁻ ein Anion ist und vorzugsweise in einer Konzentration im Bereich von 0,01 bis 5 Gew.%, bezogen auf die gesamte Zusammensetzung, ausgenommen Treibmittel, falls vorhanden, umfasst ist.

6. Zusammensetzung gemäss einem der vorhergehenden Ansprüche, **dadurch gekennzeichnet, dass** sie zusätzlich ein oder mehrere andere filmbildende Polymer(e), ausgewählt aus anionischen, nicht-ionischen, kationischen und amphoteren, umfasst.

7. Zusammensetzung gemäss einem der vorhergehenden Ansprüche, **dadurch gekennzeichnet, dass** sie ein oder mehrere synthetische oder natürliche Öl(e) umfasst.

8. Zusammensetzung gemäss einem der vorhergehenden Ansprüche, **dadurch gekennzeichnet, dass** sie ein oder mehrere Tensid(e), ausgewählt aus anionischen, nicht-ionischen, kationischen und amphoteren, umfasst.

9. Zusammensetzung gemäss einem der vorhergehenden Ansprüche, **dadurch gekennzeichnet, dass** sie ein oder mehrere Polyol(e) umfasst.

10. Zusammensetzung gemäss einem der vorhergehenden Ansprüche, **dadurch gekennzeichnet, dass** sie einen oder mehrere UV-Filter umfasst.

11. Zusammensetzung gemäss einem der vorhergehenden Ansprüche, **dadurch gekennzeichnet, dass** sie einen oder mehrere Direktfarbstoff(e) umfasst.

12. Zusammensetzung gemäss einem der vorhergehenden Ansprüche, **dadurch gekennzeichnet, dass** sie ein oder mehrere Treibmittel, vorzugsweise in einer Konzentration von 5 bis 60 Gew.%, bezogen auf die gesamte Zusammensetzung, und vorzugsweise ausgewählt aus n-Butan, Isobutan, Propan, Dimethylether, 1,1-Difluorethan und deren Mischung, umfasst.

13. Verwendung der Zusammensetzung, wie in einem der Ansprüche 1 bis 12 definiert, zum Haar-Styling, vorzugsweise Haar-Styling mit einem Eisen bei einer Oberflächentemperatur im Bereich von 130 bis 230°C.

14. Verfahren zur Haarbehandlung, wobei eine Zusammensetzung, wie in einem der Ansprüche 1 bis 12 definiert, auf das Haar aufgebracht wird und das Haar mit einem Eisen mit einer Oberflächentemperatur im Bereich von 130 bis 230°C gestylt wird.

15. Kit für Haare, umfassend eine oder mehrere Zusammensetzung(en) und gegebenenfalls ein oder mehrere Werkzeug(e), vorzugsweise mit Heizfunktion, wobei es eine Zusammensetzung, wie in einem der Ansprüche 1 bis 12 definiert, umfasst.

## Revendications

1. Composition aqueuse, aqueuse et alcoolique ou alcoolique pour cheveux, **caractérisée en ce qu'**elle comprend au moins un copolymère d'acrylate avec une température de transition vitreuse (Tg) dans la plage de 80 à 120 °C mesurée avec un calorimètre à balayage différentiel (DSC) à une cadence de chauffage de 2 °C/min, le copolymère d'acrylate étant choisi parmi
- les terpolymères de méthacrylate de butyle, d'acide méthacrylique et d'acrylate d'éthyle ; et
- les tétrapolymères de méthacrylate de butyle, d'acide méthacrylique, d'acrylate d'éthyle et de méthacrylate d'éthyle ;
et un polymère de silicone greffé avec des unités de polyéthyloxazoline.

2. Composition selon la revendication 1, **caractérisée en ce qu'**elle comprend au moins un copolymère d'acrylate en concentration dans la plage de 0,1 à 20%, de préférence 0,2 à 15, plus de préférence de 0,25 à 12,5 et le plus de préférence de 0,5 à 10 % en poids calculée sur la composition totale à l'exclusion d'un propulseur, s'il y en a.

3. Composition selon les revendications 1 et 2, **caractérisée en ce que** le copolymère d'acrylate a une Tg dans la plage de 85 à 115 °C, de préférence de 90 à 110 °C, le plus de préférence de 95 à 110 °C, mesurée avec un calorimètre à balayage différentiel (DSC) à une cadence de chauffage de 2 °C/min.

4. Composition selon l'une quelconque des revendications précédentes, **caractérisée en ce qu'**au moins un copolymère d'acrylate est un tétrapolymère de méthacrylate de butyle, d'acide méthacrylique, d'acrylate d'éthyle et de méthacrylate d'éthyle avec une température de transition vitreuse dans la plage de 98 à 108 °C mesurée avec un calorimètre à balayage différentiel (DSC) à une cadence de chauffage de 2 °C/min.

5. Composition selon l'une quelconque des revendications précédentes, **caractérisée en ce que** le polymère de silicone greffé avec des unités de polyéthyloxazoline a pour formule : 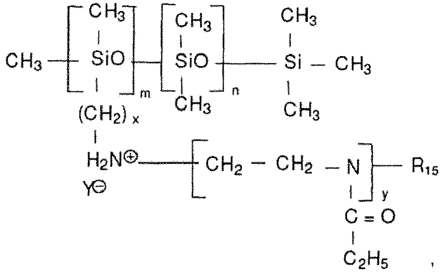 dans laquelle m et n sont chacun des nombres de 20 à 10 000, en particulier de 50 à 7 000, tout spécialement de 100 à 5 000, x est un nombre compris entre 1 et 5, de préférence 3, et y est un nombre de 5 à 30, R₁₅ est un groupement alkyle ou aryle en C₁-C₁₂, en particulier un groupement méthyle, éthyle ou benzyle et Y- est un anion, et de préférence compris à une concentration dans la plage de 0,01 à 5 % en poids, calculée sur la composition totale à l'exclusion d'un propulseur, s'il y en a.

6. Composition selon l'une quelconque des revendications précédentes, **caractérisée en ce qu'**elle comprend en outre un ou plusieurs polymères filmogènes choisis parmi des polymères anioniques, non-ioniques, cationiques et amphotères.

7. Composition selon l'une quelconque des revendications précédentes, **caractérisée en ce qu'**elle comprend une ou plusieurs huiles synthétiques ou naturelles.

8. Composition selon l'une quelconque des revendications précédentes, **caractérisée en ce qu'**elle comprend un ou plusieurs tensioactifs choisis parmi les tensioactifs anioniques, non-ioniques, cationiques et amphotères.

9. Composition selon l'une quelconque des revendications précédentes, **caractérisée en ce qu'**elle comprend un ou plusieurs polyols.

10. Composition selon l'une quelconque des revendications précédentes, **caractérisée en ce qu'**elle comprend un ou plusieurs filtres UV.

11. Composition selon l'une quelconque des revendications précédentes, **caractérisée en ce qu'**elle comprend un ou plusieurs colorants directs.

12. Composition selon l'une quelconque des revendications précédentes, **caractérisée en ce qu'**elle comprend un ou plusieurs propulseurs, de préférence en concentration de 5 à 60 % en poids calculée sur la composition totale et de préférence choisis parmi le n-butane, l'isobutane, le propane, le diméthyléther, le 1,1-difluoroéthane et leurs mélanges.

13. Utilisation de la composition selon l'une quelconque des revendications 1 à 12 pour la mise en forme des cheveux, de préférence pour la mise en forme de cheveux avec un fer à une température de surface dans la plage de 130 à 230 °C.

14. Procédé de traitement capillaire, dans lequel une composition telle que définie dans l'une quelconque des revendications 1 à 12 est appliquée sur les cheveux et les cheveux sont mis en forme avec un fer à une température de surface dans la plage de 130 à 230 °C.

15. Kit capillaire comprenant une ou plusieurs compositions et éventuellement un ou plusieurs outils, de préférence avec une capacité de chauffage, dans lequel il comprend une composition telle que définie dans l'une quelconque des revendications 1 à 12.
